# EUROPÄISCHE PATENTSCHRIFT

(11) **EP 1 599 594 B1**
(45) Veröffentlichungstag und Bekanntmachung des Hinweises auf die Patenterteilung: **30.05.2007**
(21) Anmeldenummer: 04705779.9
(22) Anmeldetag: 28.01.2004
(51) Int. Cl.: C12P 7/04, C12P 7/06, C12P 7/62, C12P 17/18

(54) **ENZYMATISCHE HERSTELLUNG VON (METH)ACRYLSÄUREESTERN**
ENZYMATIC PRODUCTION OF (METH)ACRYLIC ACID ESTERS
PRODUCTION ENZYMATIQUE D'ESTERS D'ACIDE (METHYLE)ACRYLIQUE

(30) Priorität: 26.02.2003 DE 10308504
(43) Veröffentlichungstag der Anmeldung: 30.11.2005
(73) Patentinhaber: BASF Aktiengesellschaft, 67056 Ludwigshafen (DE)
(72) Erfinder: HÄRING, Dietmar, 69198 Schriesheim (DE); WAGNER, Eva, 67346 Speyer (DE); BRUCHMANN, Bernd, 67251 Freinsheim (DE); BECK, Erich, 68526 Ladenburg (DE); HAUER, Bernhard, 67136 Fussgönheim (DE)
(86) Internationale Anmeldenummer: PCT/EP2004/000700
(87) Internationale Veröffentlichungsnummer: WO 2004/076676

(56) Entgegenhaltungen:
- WO-A-00/63150
- WO-A-01/46286
- DE-A- 10 118 232
- DE-A- 19 647 395
- US-A- 2 680 735
- US-A- 5 240 835
- DATABASE WPI Section Ch, Week 199248 Derwent Publications Ltd., London, GB; Class A25, AN 1992-394000 XP002282215 & JP 04 292675 A (ASAHI CHEM IND CO LTD), 16. Oktober 1992 (1992-10-16)
- TOR R ET AL: "Enzymatically catalysed transesterifications of acryl and methacryl mo" ENZYME AND MICROBIAL TECHNOLOGY, Bd. 12, Nr. 1, 1990, Seiten 299-304, XP002138281 ISSN: 0141-0229
- KUMAR R ET AL: "Biocatalytic route to well-defined macromers built around a sugar core" JOURNAL OF THE AMERICAN CHEMICAL SOCIETY, Bd. 124, Nr. 9, 6. März 2002 (2002-03-06), Seiten 1850-1851, XP002282214 ISSN: 0002-7863 in der Anmeldung erwähnt
- KUMAR R ET AL: "Enzyme-catalyzed synthesis of well-defined macromers built around a sugar core" ACS SYMPOSIUM SERIES, Bd. 840, 2003, Seiten 107-118, XP008031056
- VÄNTTINEN E ET AL: "Optically Active 1,3-Dioxolane-4-methanols. Lipase-Catalysed Acylation of Racemic Ketals and Diastereomeric Acetals" TETRAHEDRON: ASYMMETRY, Bd. 7, Nr. 10, 1. Oktober 1996 (1996-10-01), Seiten 3037-3046, XP004048392 ISSN: 0957-4166
- HAJJAR A B ET AL: "Preparation of monomeric acrylic ester intermediates using lipase catalysed transesterifications in organic solvents" BIOTECHNOLOGY LETTERS, Bd. 12, Nr. 11, 1990, Seiten 825-830, XP008031024 ISSN: 0141-5492 in der Anmeldung erwähnt
- MORI H ET AL: "Protection and polymerization of functional monomers. 21. Anionic living polymerization of (2,2-dimethyl-1,3-dioxolan-4-yl)methylmeth acrylate" MACROMOLECULES, Bd. 27, 1994, Seiten 35-39, XP002282411

## Beschreibung

Die vorliegende Erfindung betrifft ein Verfahren zur Herstellung von teilveresterten (Meth)acrylsäureestern von Polyalkoholen und deren Verwendung.

Die Herstellung von (Meth)acrylsäureestern erfolgt zumeist durch säure-oder basenkatalysierte Veresterung von (Meth)acrylsäure oder Umesterung von anderen (Meth)-acrylsäureestern mit Alkoholen.

Teilveresterte (Meth)acrylsäureester lassen sich durch eine Ver- oder Umesterung in der Regel nicht gezielt herstellen, da statistische Gemische erhalten werden.

Bei Verwendung acetal- oder ketalgeschützten Polyalkoholen ist ein säurekatalysiertes Verfahren zu deren Herstellung nicht mehr möglich, da Acetal- und Ketalgruppen säurelabil sind.

Bei basenkatalysierter Umesterung oder anderen Synthesen entstehen oft komplexe und bisweilen gefärbte Produktgemische. Zur Entfernung von Färbungen und unumgesetzten Reaktanden müssen die Produktgemische durch aufwendige alkalische Wäschen aufgearbeitet werden.

US 2 680 735 beschreibt die Herstellung von (Meth)acrylsäureestern von acetal- und ketalgeschütztem Glycerin durch eine Umesterung des acetal- und ketalgeschütztem Glycerin mit niederen (Meth)acrylsäureestern bei Temperaturen oberhalb von 70 °C und Katalyse von Alkalimetalloxiden, Natriummethylat oder Natriumethylat.

R. Deschenaux und J. K. Stille beschreiben in *J. Org. Chem.,* **1985,** *50*, 2299 - 2302 die gleiche Reaktion unter Verwendung von Titantetraisopropylat als Katalysator und anschließender saurer Spaltung der Ketalschutzgruppen.

Nachteilig an dieser Herstellvariante ist, daß bei Verwendung von Metallkatalysatoren Färbungen des Reaktionsgemischs auftreten können.

JP 2001-294 555 (CA-Nr. 135:303 607) und JP 2001-294 554 (CA-Nr. 135:303 608) beschreiben die Herstellung von Glycerinmonomethacrylat durch Umsetzung von Methacrylsäure mit Glycidol (2,3-Epoxy-1-propanol). Nachteilig dabei ist die Ausbeute von nicht mehr als 80 % und die geringe Reinheit von 95%.

JP 08-277 245 (CA-Nr. 126:31792) beschreibt die Herstellung von Glycerinmono-(meth)acrylat durch Hydrolyse von Glycidyl(meth)acrylat.

Nachteilig an diesen Syntheserouten ist jedoch der Umgang mit hochreaktiven und toxischen Epoxiden.

Die Herstellung von (Meth)acrylsäureestern durch eine enzymatische Ver- oder Umesterung ist bekannt.

Kumar und Gross beschreiben in *J. Am. Chem. Soc.* **2002**, *124*, 1850-1851 die Lipasekatalysierte Umsetzung von Isopropyliden-geschützten Zuckern durch Umsetzung mit Vinylmethacrylat. Eine vollständige Umsetzung wird erreicht durch das spezielle Edukt Vinylmethacrylat, da freigesetzter Vinylalkohol dem Reaktionsgleichgewicht als Acetaldehyd entzogen wird. Nachteilig an diesem Verfahren ist, daß Vinylmethacrylat nicht kommerziell verfügbar ist.

A. T. J. W. de Goede et al. beschreiben in *Biocatalysis,* **1994**, *9*, 145-155 die Umesterung von α-O-Octyl-Glucosid mit Ethylacrylat zum 6-O-Acrylester in Gegenwart von Lipasen. Nachteilig an diesem Verfahren ist, daß es auf Glucoside und glykosidische Bindungen beschränkt ist und empfindlich auf sterische Einflüsse im Glucosid reagiert. Zudem wurden höher acrylierte Produkte infolge von unselektiven Nebenreaktionen erhalten.

EP-A1 999 229 beschreibt die enzymatische Ver- und Umesterung von Polyoxyalkylenen.

Hajjar et al. beschreiben in *Biotechnol. Lett.* **1990**, *12*, 825-830 die enzymatische Umesterung von cyclischen und offenkettigen Alkandiolen mit Ethylacrylat mit einer Lipase aus *Chromobacteriurn viscosum.* Die Reaktionen laufen bei einem 18-fachen molaren Überschuß des Alkylacrylats gegenüber dem Diol in einem lösungsmittelfreien System ab. Es entstehen Mischungen aus Mono- und Diacrylaten.

US 5240835 beschreibt die Umesterung von Alkylacrylaten mit Alkoholen unter Katalyse eines Biokatalysators aus *Corynebacterium oxydans.* Beispielhaft wird dort die Reaktion von einem 96-fachen molaren Überschuß Ethylacrylat mit 2,2-Dimethyl-1,3-propandiol aufgeführt. Lediglich 21 % Ausbeute wurden nach 3 Tagen bei 30 °C erhalten.

Aufgabe der vorliegenden Erfindung war es, ein weiteres Verfahren zur Verfügung zu stellen, mit dem teilveresterte (Meth)acrylsäureester von Polyalkoholen in hohen Umsätzen und hohen Reinheiten aus einfachen Edukten herstellbar sind. Die Synthese sollte unter milden Bedingungen ablaufen, so daß Produkte mit einer niedrigen Farbzahl und hoher Reinheit resultieren.

Die Aufgabe wurde gelöst durch ein Verfahren zur Herstellung von teilveresterten (Meth)acrylsäureestern dadurch gekennzeichnet, daß man in einem Schritt c) Polyalkohole (C), in denen mindestens zwei Hydroxygruppen gemeinsam in einer Acetal- oder Ketalgruppe verbunden sind,
in Gegenwart mindestens eines Enzyms (E) mit (Meth)acrylsäure verestert oder
in Gegenwart mindestens eines Enzyms (E) mit mindestens einem (Meth)acrylat (D) umestert, wobei das (Meth)acrylat (D) ein Ester von (Meth)acrylsäure mit einem gesättigten Alkohol ist.

Mit Hilfe des erfindungsgemäßen Verfahrens ist die Herstellung teilveresterten (Meth)-acrylsäureester (F) in hoher chemischer und Raum-Zeit-Ausbeute und unter milden. Bedingungen mit guten Farbzahlen möglich.

(Meth)acrylsäure steht in dieser Schrift für Methacrylsäure und Acrylsäure, bevorzugt für Acrylsäure.

Erfindungsgemäß einsetzbare Polyalkohole (C) sind solche Verbindungen, die mindestens eine Acetal- oder Ketalgruppe, bevorzugt 1 bis 5, besonders bevorzugt 1 bis 3. ganz besonders bevorzugt 1 bis 2 und insbesondere eine Acetal- oder Ketalgruppe, sowie mindestens eine Hydroxygruppe (-OH), bevorzugt 1 bis 10, besonders bevorzugt 1 bis 6, ganz besonders bevorzugt 1 bis 3, insbesondere 1 bis 2 und speziell eine Hydroxygruppe enthalten.

In den Acetal- und Ketalgruppen der Polyalkohole (C) sind jeweils 2 Hydroxygruppen der den Polyalkoholen (C) zugrundeliegenden Polyole (B) gemeinsam verbunden.

Bevorzugte Polyalkohole (C) sind solche, die erhältlich sind durch
(a) Umsetzung eines Aldehyds oder Ketons (A) mit einem Polyol (B) und
(b) gegebenenfalls Aufreinigung des aus a) erhältlichen Reaktionsgemisches.

Aldehyde oder Ketone (A) sind solche der Formel I,

R¹-C(=O)-R² (I),

worin
R¹ und R² unabhängig voneinander Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen. Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können oder R¹ und R² gemeinsam mit dem Carbonylkohlenstoff einen vier- bis zwölfgliedrigen Ring bilden können.

Beispiele für R¹ und/oder R² sind Wasserstoff, Methyl, Ethyl, *iso*-Propyl, n-Propyl, n-Butyl, *iso*-Butyl, *sek-*Butyl, *tert*-Butyl, n-Hexyl, n-Heptyl, n-Octyl, n-Decyl, n-Dodecyl, n-Tetradecyl, n-Hexadecyl, n-Octadecyl, 2-Ethylhexyl, Cyclopentyl, Cyclohexyl, Cyclooctyl, Cyclododecyl, Phenyl oder Naphthyl, bevorzugt sind Wasserstoff, Methyl und Phenyl, besonders bevorzugt ist Methyl.

Beispiele für Aldehyde und Ketone (A) sind Formaldehyd, Acetaldehyd, Propionaldehyd, n-Butyraldehyd, iso-Butyraldehyd, Pivaldehyd, Benzaldehyd, Aceton, Methylethylketon, Diethylketon, Acetophenon, Benzophenon, Cyclopentanon, Cyclohexanon oder Cyclododekanon, bevorzugt sind Formaldehyd, Acetaldehyd, Pivaldehyd, Aceton, Methylethylketon, Diethylketon, Cyclopentanon oder Cyclohexanon, besonders bevorzugt sind Formaldehyd, Aceton, Cyclopentanon und ganz besonders bevorzugt ist Aceton.

Polyole (B) sind Polyole mit 3 bis 10, bevorzugt 3 bis 6 Hydroxygruppen.

Beispiele für Polyole (B) sind Glycerin, Diglycerin, Trimethylolbutan, Trimethylolpropan, Ditrimethylolpropan, Trimethylolethan, Pentaerythrit, Dipentaerythrit, Sorbit, Mannit, Diglycerol, Threit, Erythrit, Adonit (Ribit), Arabit (Lyxit), Xylit, Dulcit (Galactit), Maltit oder Isomalt. Bevorzugt sind Sorbit, Glycerin, Diglycerin, Trimethylolpropan, Ditrimethylolpropan, Trimethylolethan und Pentaerythrit, besonders bevorzugt sind Glycerin, Trimethylolpropan und Pentaerythrit und ganz besonders bevorzugt ist Glycerin. Die Umsetzung eines Aldehyds oder Ketons (A) mit einem Polyol (B) ist an sich bekannt und nicht beschränkt und kann beispielsweise erfolgen, wie beschrieben in Organikum, VEB Deutscher Verlag der Wissenschaften, 17. Auflage, Berlin 1988, S. 398.

Typischerweise werden der Aldehyd/das Keton (A) und das Polyol (B) in einer Stöchiometrie von 0,7 bis 1,2 mol (A): 2 mol zu schützender Hydroxygruppen in (B), bevorzugt 0,8 -1,2 : 2, besonders bevorzugt 0,9 -1,1:2, ganz besonders bevorzugt 0,95 - 1,1:2 und insbesondere 1:2 mol/mol miteinander umgesetzt.

Die Umsetzung erfolgt in der Regel bei einer Temperatur von 0 bis 120 °C, besonders bei 20 bis 100, ganz besonders bevorzugt 30 bis 80 und ganz besonders bevorzugt 40 bis 80 °C.

Die Umsetzung ist in der Regel innerhalb von 12 Stunden beendet, bevorzugt innerhalb von 15 Minuten bis 10 Stunden, besonders bevorzugt in 30 Minuten bis 8 Stunden, ganz besonders bevorzugt 45 Minuten bis 6 Stunden und insbesondere innerhalb von 1 bis 4 Stunden.

Die Acetalisierung/Ketalisierung erfolgt in der Regel säurekatalysiert, beispielsweise katalysiert durch Mineralsäuren, wie beispielsweise Salzsäure, Schwefelsäure, Salpetersäure oder Phosphorsäure, para-Toluolsulfonsäure, Benzolsulfonsäure, Methansulfonsäure, Trifluormethansulfonsäure, mineralische Tone, saure Ionentauscher oder katalysiert durch Enzyme.

Die Umsetzung kann ohne Lösungsmittel durchgeführt werden oder in bevorzugt Anwesenheit eines solchen, beispielsweise Ether, Kohlenwasserstoffe oder halogenierte Kohlenwasserstoffe.

Bevorzugt wird die Acetalisierung/Ketalisierung unter Entfernung des bei der Reaktion freigesetzten Wassers durchgeführt, beispielsweise in Gegenwart von Molekularsieb oder Zeolithen oder durch Membrantrennung oder besonders bevorzugt unter azeotroper Entfernung von Wasser durch ein Lösungsmittel, das ein Azeotrop mit Wasser bildet.

Besonders bevorzugt kann die Acetalisierung/Ketalisierung erfolgen, wie in DE-A1 196 47 395 und dort besonders von S. 2, Z. 55 bis S. 4, Z. 20 beschrieben, auf deren Offenbarung hiermit ausdrücklich Bezug genommen wird.

Das aus a) erhältliche Reaktionsgemisches kann in einem weiteren Schritt b) falls gewünscht aufgereinigt werden, beispielsweise durch Filtration, Destillation, Rektifikation, Chromatographie, Behandlung mit lonentauschem, Adsorbentien, neutraler, saurer und/oder alkalischer Wäsche, Strippen oder Kristallisation.

Somit sind Beispiele für die erfindungsgemäß einsetzbaren acetal- oder ketalgeschützten Polyalkohole (C) solche Polyalkohole, die durch Schützen der Polyole (B) mit Aldehyden oder Ketonen (A) herstellbar sind.

Dies sind besonders bevorzugt solche Polyalkohole (C), die eine in Position 2 mono- oder disubstituierte 1,3-Dioxolan- oder 1,3-Dioxanstruktur enthalten, ganz besonders bevorzugt solche, die eine 2,2-Dimethyl-1,3-dloxolan- oder eine 2,2-Dimethyl-1,3-dioxanstruktur enthalten.

Insbesondere bevorzugt sind als Polyalkohole (C) 4-Hydroxymethyl-2,2-dimethyl-1,3-diaxolan, 4-Hydroxymethyl-2-methyl-1,3-dioxolan, 4-Hydroxymethyl-2,2-diethyl-1,3-dioxolan, 4-Hydroxymethyl-2-*tert*-butyl-1,3-dioxolan, 4-Mydroxymethyl-2-phenyl-1,3-dioxoian, 5-Ethyl-5-hydroxymethyl-2,2-dimethyl-1,3-dioxan, 5-Ethyl-5-hydroxymethyl-2-methyl-1,3-diaxan, 5-Ethyl-5-hydroxymethyl-2,2-diethyl-1,3-dioxan, 5-Ethyl-5-hydroxymethyl-2-tert-butyl-1,3-dioxan, 5-Ethyl-5-hydroxymethyl-2-phenyl-1,3-dioxan, 1,2-O-Isopropyliden-a-D-glucofuranose, 2,3-O-Isopropyliden-threitol (= 2,2-Dimethyl-1,3-dipxolan-4,5-dirnethanol) und 5,5-Bis(hydroxymethyl)-2,2-dimethyl-1,3-dioxan. Ein weiteres Beispiel ist 5-Ethyl-5-hydroxymethyl-1,3-dioxan.

In Schritt c) erfolgt die Veresterung mit (Meth)acrylsäure oder bevorzugt die Umesterung des acetal- oder ketalgeschützten Polyalkohols (C) mit mindestens einem, bevorzugt einem (Meth)acrylat (D) in Anwesenheit mindestens eines, bevorzugt eines die Umesterung katalysierenden Enzyms (E).

Verbindungen (D) können (Meth)acrylsäure oder Ester von (Meth)acrylsäure mit einem gesättigten Alkohol sein, bevorzugt gesättigte C₁ - C₁₀-Alkylester der (Meth)acrylsäure, besonders bevorzugt gesättigte C₁ - C₄-Alkylester der (Meth)acrylsäure.

Gesättigt bedeutet im Rahmen dieser Schrift Verbindungen ohne C-C-Mehrfachbindungen (außer selbstverständlich die C=C-Doppelbindung in den (Meth)acryleinheiten).

Beispiele für Verbindungen (D) sind (Meth)acrylsäuremethyl-, -ethyl-, -n-butyl-, *-iso-*butyl-, n-octyl- und -2-Ethylhexylester, 1,2-Ethylenglycolmono(meth)acrylat, 1,4-Butandiolmono(meth)acrylat und 1,6-Hexandiolmono(meth)acrylat.

Besonders bevorzugt sind (Meth)acrylsäuremethyl-, -ethyl-, -n-butyl- und -2-Ethylhexylester und ganz besonders bevorzugt (Meth)acrylsäuremethyl-, -ethyl- und -n-butylester.

Die enzymatische Ver- oder Umesterung mit einem (Meth)acrylat erfolgt im Allgemeinen bei 0 bis 100°C, bevorzugt 20 bis 80 °C, besonders bevorzugt 20 bis 70°C, ganz besonders bevorzugt 20 bis 60 °C.

Erfindungsgemäß einsetzbare Enzyme (E) sind beispielsweise ausgewählt unter Hydrolasen (E.C. 3.-.-.-), und unter diesen besonders unter den Esterasen (E.C. 3.1.-.-), Lipasen (E.C. 3.1.1.3), Glykosylasen (E.C. 3.2.-.-) und Proteasen (E.C. 3.4.-.-) in freier oder auf einem Träger chemisch oder physikalisch immobilisierter Form, bevorzugt Lipasen, Esterasen oder Proteasen und besonders bevorzugt Esterasen (E.C. 3.1.-.-). Ganz besonders bevorzugt sind Novozyme 435 (Lipase aus *Candida antarctica* B) oder Lipase aus Aspergillus sp., Aspergillus niger sp., Mucor sp., Penicilium cyclopium sp., Geotricum candidum sp., Rhizopus javanicus, Burkholderia sp., Candida sp., Pseudomonas sp., oder Schweinepankreas, insbesondere bevorzugt sind Lipase aus *Candida antarctica* B oder aus Burkholderia sp.

Der Enzymgehalt im Reaktionsmedium liegt in der Regel im Bereich von etwa 0,1 bis 10 Gew.%, bezogen auf die Summe der eingesetzten Komponenten (C) und (D).

Die Reaktionszeit hängt unter anderem von der Temperatur, der verwendeten Menge und der Aktivität des Enzymkatalysators und vom geforderten Umsatz ab sowie vom teilveresterten Alkohol. Bevorzugt wird die Reaktionszeit so angepaßt, daß der Umsatz aller ursprünglich im Alkohol (C) enthaltenden Hydroxyfunktionen mindestens 70%, bevorzugt mindestens 80, besonders bevorzugt mindestens 90, ganz besonders bevorzugt mindestens 95 % und insbesondere mindestens 97 % beträgt. In der Regel sind dafür 1 bis 48 Stunden, bevorzugt 1 bis 12 und besonders bevorzugt 1 bis 6 Stunden ausreichend.

Das molare Verhältnis von (Meth)acrylsäureverbindung (D) (bezogen auf die (Meth)acryleinheiten) zu teilveresterten Alkohol (C) (bezogen auf Hydroxygruppen) kann in einem weiten Bereich, wie z.B. im Verhältnis 100:1 bis 1:1, bevorzugt 50:1 bis 1:1, besonders bevorzugt 20:1 bis 1:1 und ganz besonders bevorzugt 10:1 bis 1:1, schwanken.

Die Reaktion kann in organischen Lösungsmitteln oder deren Gemischen oder ohne Zusatz von Lösungsmitteln ablaufen. Die Ansätze sind in der Regel weitgehend wasserfrei (d.h. unter 10, bevorzugt unter 5, besonders bevorzugt unter 1 und ganz besonders bevorzugt unter 0,5 Vol% Wasserzusatz).

Geeignete organische Lösungsmittel sind solche für diese Zwecke bekannten, beispielsweise tertiäre Monoole, wie C₃-C₆-Alkohole, bevorzugt tert-βutanol, tert-Amylalkohol, Pyridin, Poly-C₁-C₄-alkylenglykoldi-C₁-C₄-alkylether, bevorzugt Polyethylenglycoldi-C₁-C₄-alkylether, wie z.B. 1,2-Dimethoxy.ethan, Diethylenglycoldimethylether, Polyethylenglycoldimethylether 500, C₁-C₄-Alkylencarbonate, insbesondere Propylencarbonat, C₃-C₆-Alkylessigsäureester, insbesondere tert.-Butyl-essigsäureester, THF, Toluol, 1,3-Dioxolan, Aceton, iso-Butyl-methylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen. Es kann vorteilhaft sein, freiwerdendes Wasser oder Alkohol durch ein möglichst nahe am Temperaturoptimum des verwendeten Enzyms siedendes binäres oder ternäres Heteroazeotrop abzutrennen. Der so entfernte Alkohol kann dann durch Phasenscheidung oder Membrandampftrennung entfernt werden.

Wahlweise können zu den organischen Lösungsmitteln wässrige Lösungsmittel zugesetzt werden, so dass - je nach organischem Lösungsmittel - ein- oder mehrphasige Reaktionslösungen entstehen. Beispiele für wässrige Lösungsmittel sind Wasser sowie wässrige, verdünnte (z.B. 10 bis 100mM) Puffer, beispielsweise mit einem pH-Wert im Bereich von etwa 6 bis 8, wie z.B. Kaliumphosphat- oder TRIS-HCl-Puffer.

Der Wasseranteil im Reaktionsansatz liegt in der Regel bei 0-10 Vol%. Bevorzugt werden die Reaktanden ohne Vorbehandlung (Trocknung, Wasserdotierung) eingesetzt.

Die Substrate liegen entweder gelöst, als Feststoffe suspendiert oder in Emulsion im Reaktionsmedium vor. Vorzugsweise liegt die anfängliche Konzentration der Reaktanden im Bereich von etwa 0,1 bis 20 Mol/l, insbesondere bei 0,15 bis 10 Mol/l oder 0,2 bis 5 mol/l liegt.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt und zum Start der Reaktion, sowie gegebenenfalls ein- oder mehrmals im Verlauf der Reaktion, mit dem Enzympräparat versetzt. Die Temperatur wird während der Reaktion auf den gewünschten Wert eingestellt und kann, falls gewünscht, während des Reaktionsverlauf erhöht oder verringert werden.

Wird die Reaktion im Festbettreaktor durchgeführt, so ist der Festbettreaktor bevorzugt mit immobilisierten Enzymen bestückt, wobei die Reaktionsmischung durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen, wobei auch unter Vakuum der freigesetzte Alkohol gleichzeitig abdestilliert werden kann.

Die Entfernung von Wasser im Falle einer Veresterung oder Alkoholen, die bei einer Umesterung aus den Alkyl(meth)acrylaten freigesetzt werden, erfolgt kontinuierlich oder schrittweise in an sich bekannter Weise, z.B. durch Vakuum, azeotrope Entfernung, Absorption, Pervaporation und Diffusion über Membranen.

Hierzu eignen sich vorzugsweise Molekularsiebe oder Zeolithe (Porengröße z.B. im Bereich von etwa 3-10 Angström), eine Abtrennung durch Destillation oder mit Hilfe geeigneter semipermeabler Membranen.

Es ist aber auch möglich, das abgetrennte Gemisch aus Alkyl(meth)acrylat und dem diesem zugrundeliegenden Alkohol, das häufig ein Azeotrop bildet, direkt in eine Anlage zur Herstellung des Alkyl(meth)acrylats zuzuführen, um es dort in einer Veresterung mit (Meth)acrylsäure wiederzuverwerten.

Nach Beendigung der Reaktion kann man das aus c) erhältliche Reaktionsgemisch ohne weitere Aufreinigung weiterverwenden oder es erforderlichenfalls in einem weiteren Schritt d) aufreinigen. ,

d) In der Regel wird lediglich das eingesetzte Enzym vom Reaktionsgemisch abgetrennt und das Reaktionsprodukt vom gegebenenfalls verwendeten organischen Lösungsmittel abgetrennt.

Eine Abtrennung vom Enzym erfolgt in der Regel durch Filtration, Absorption, Zentrifugation oder Dekantieren. Das abgetrennte Enzym kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Zur weiteren Aufreinigung des Reaktionsproduktes kann auch eine Chromatographie durchgeführt werden.

Bevorzugt werden in Schritt d) jedoch lediglich das eingesetzte Enzym und das gegebenenfalls eingesetzte Lösungsmittel abgetrennt.
Die Reaktionsbedingungen bei der enzymatischen Ver- oder Umesterung sind mild. Aufgrund der niedrigen Temperaturen und sonstigen milden Bedingungen wird die Bildung von Nebenprodukten in Schritt c) vermieden, die andernfalls zum Beispiel von chemischen Katalysatoren stammen können oder durch unerwünschte radikalische Polymerisation des eingesetzten (Meth)acrylats, die sonst nur durch Zugabe von Stabilisatoren verhindert werden kann. Bei der erfindungsgemäßen Reaktionsführung kann der (Meth)acrylverbindung (D) über den ohnehin enthaltenen Lagerstabilisator hinaus zusätzliche Stabilisator zugegeben werden, beispielsweise Hydrochinonmonomethylether, Phenothiazin, Phenole, wie z.B. 2-tert.-Butyl-4-methylphenol, 6-tert.-Butyl-2,4-dimethyl-phenol oder N-Oxyle, wie 4-Hydroxy-2,2,6,6-tetramethyl-piperidin-N-oxyl, 4-Oxo-2,2,6,6-tetramethyl-piperidin-N-oxyl, beispielsweise in Mengen von 50 bis 2000 ppm. Vorteilhaft wird die Ver- oder Umesterung in Gegenwart eines sauerstoffhaltigen Gases, bevorzugt Luft oder Luft-Stickstoff-Gemische, durchgeführt. Des weiteren kann der Enzymkatalysator unproblematisch vom Endprodukt entfernt werden. Des weiteren wird in der Regel keine wesentliche Spaltung der Acetal- oder Ketalgruppen durch enzymatische Verseifung festgestellt, man findet in der Regel weniger als 10 %, bevorzugt weniger als 5 % Nebenprodukte.

Die aus den Stufen c) beziehungsweise d) erhältlichen acetal- beziehungsweise ketalgeschützten (Meth)acrylsäureester können bereits als solche verwendet werden, werden jedoch bevorzugt in einer Stufe e) entschützt und gegebenenfalls in einer Stufe f) aufgereinigt.

Die Spaltung in Stufe e) erfolgt in der Regel säurekatalysiert und unter Zugabe von Wasser und ist an sich bekannt und nicht beschränkt.

Die Umsetzung erfolgt in der Regel bei einer Temperatur von 20 bis 150 °C, besonders bei 40 bis 120 und ganz besonders bevorzugt 50 bis 100 °C.

Die Umsetzung ist in der Regel innerhalb von 12 Stunden beendet, bevorzugt innerhalb von 15 Minuten bis 10 Stunden, besonders bevorzugt in 30 Minuten bis 8 Stunden, ganz besonders bevorzugt 45 Minuten bis 6 Stunden und insbesondere innerhalb von 1 bis 4 Stunden.

Die Spaltung der Acetale/Ketale erfolgt in der Regel säurekatalysiert, beispielsweise katalysiert durch Mineralsäuren, wie beispielsweise Salzsäure, Schwefelsäure, Salpetersäure oder Phosphorsäure, para-Toluolsulfonsäure, Benzolsulfonsäure, Methansulfonsäure, Trifluormethansulfonsäure, mineralische Tone, saure Ionentauscher oder katalysiert durch Enzyme unter Zugabe von bis zu 20, bevorzugt bis zu 15 und besonders bevorzugt bis zu 10 Gew% Wasser.
Die Umsetzung kann ohne Lösungsmittel durchgeführt werden oder in bevorzugt Anwesenheit eines solchen, beispielsweise Ether, Alkohole, Kohlenwasserstoffe, Ketone, halogenierte Kohlenwasserstoffe oder Wasser.

Die Spaltung kann bevorzugt durchgeführt werden wie in WO 00/63149, dort besonders von S. 4, Z. 28 bis S. 18, Z. 24 und den Beispielen, WO 00/63150, dort besonders von S. 5, Z. 12 bis S. 16, Z. 30 und den Beispielen sowie in DE-A1 101 18 232, dort besonders von S. 3, Z. 49 bis S. 4, Z. 32 und den Beispielen beschrieben, wobei auf die Offenbarung dieser drei Schriften hiermit ausdrücklich Bezug genommen wird.

Das aus e) erhältliche Reaktionsgemisches kann in einem weiteren Schritt f) falls gewünscht aufgereinigt werden, beispielsweise durch Filtration, Destillation, Rektifikation, Chromatographie, Behandlung mit lonentauschem, Adsorbentien, neutraler, saurer und/oder alkalischer Wäsche, Strippen oder Kristallisation.

Die aus den Stufen c) beziehungsweise d) erhältlichen acetal- beziehungsweise ketalgeschützten (Meth)acrylsäureester beziehungsweise die aus den Schritten e) beziehungsweise f) erhältlichen teilveresterten (Meth)acrylsäureester (F) können vorteilhaft als Monomere oder Comonomere in Poly(meth)acrylaten oder als Reaktivverdünner in strahlungshärtbaren und/oder Dual-Cure-härtbaren Poly(meth)acrylaten eingesetzt werden. Derartige Poly(meth)acrylate sind beispielsweise als Bindemittel in strahlungs- oder Dual-Cure-härtbaren Beschichtungsmitteln geeignet. Weiterhin sind die teilveresterten (Meth)acrylsäureester (F) verwendbar in Polyurethanen, beispielsweise PU-Dispersionen, PU-Schäumen, PU-Klebstoffen und PU-Beschichtungen.

So erhältliche Beschichtungen weisen sehr hohe Kratzfestigkeiten, Härten, Chemikalienbeständigkeiten, Elastizität und Haftung, sowohl auf hydrophilen als auch auf hydrophoben Substraten auf.

Ein weiterer Gegenstand der vorliegenden Anmeldung ist daher die Verwendung der nach dem erfindungsgemäßen Verfahren hergestellten acetal-lketalgeschützten oder teilveresterten (Meth)acrylsäureester als Reaktiwerdünner oder Bindemittel in Strahlen oder Dual-Cure-härtbaren Beschichtungsmassen, bevorzugt in Deckbeschichtungen, besonders bevorzugt in transparenten Klarlacken. Selbstverständlich können die erfindungsgemäß hergestellten teilveresterten (Meth)acrylsäureester auch als Monomere in Polymerisationen, gegebenenfalls zusammen mit anderen polymerisierbaren Monomeren, wie z.B. (Meth)acrylsäure, (Meth)acrylsäureester, Styrol, Butadien, Acrylnitril, Vinylacetat, N-Vinylpyrrolidon, 4-Hydroxybutylvinylether oder N-Vinylformamid, verwendet werden.

Unter "Dual-Cure" ist zu verstehen, dass die Beschichtungsmassen thermisch und mit aktinischer Strahlung härtbar sind. Im Rahmen der vorliegenden Erfindung ist unter aktinischer Strahlung elektromagnetische Strahlung wie sichtbares Licht, UV-Strahlung oder Röntgenstrahlung, insbesondere UV-Strahlung, und Korpuscularstrahlung wie Elektronenstrahlung zu verstehen.

Strahlenhärtbare Bindemittel sind solche, die mittels aktinischer Strahlung wie vorstehend definiert, insbesondere mittels UV-Strahlung härtbar sind.

Ein weiterer Gegenstand der vorliegenden Anmeldung sind Lackformulierungen, enthaltend die nach dem erfindungsgemäßen Verfahren erhältlichen acetal- /ketalgeschützten oder teilveresterten (Meth)acrylsäureester. Dabei können die teilveresterten (Meth)acrylsäureester sowohl in Basislacken als auch in Decklacken eingesetzt werden. Aufgrund ihrer besonderen Eigenschaften, wie der Erhöhung der Kratzfestigkeit und Elastizität, sowie der Erniedrigung der Viskosität, insbesondere bei verzweigten Polyacrylaten, einer strahlengehärteten Klarlackbeschichtung, ist ihr Einsatz in Deckbeschichtungen bevorzugt.

Neben den nach dem erfindungsgemäßen Verfahren erhältlichen teilveresterten (Meth)acrylsäureester (F) kann eine erfindungsgemäße strahlungshärtbare Masse noch folgende Komponenten enthalten:
(G) mindestens eine polymerisierbare Verbindung mit mehreren copolymerisierbaren, ethylenisch ungesättigten Gruppen,
(H) gegebenenfalls Reaktiwerdünner,
(I) gegebenenfalls Photoinitiator sowie
(J) gegebenenfalls weitere lacktypische Additive.

Als Verbindungen (G) kommen strahlungshärtbare, radikalisch polymerisierbare Verbindungen mit mehreren, d.h. mindestens zwei, copolymerisierbaren, ethylenisch ungesättigten Gruppen in Betracht.

Bevorzugt handelt es sich bei Verbindungen (G) um Vinylether- oder (Meth)acrylatverbindungen, besonders bevorzugt sind jeweils die Acrylatverbindungen, d.h. die Derivate der Acrylsäure.

Bevorzugte Vinylether und (Meth)acrylat-Verbindungen (G) enthalten 2 bis 20, bevorzugt 2 bis 10 und ganz besonders bevorzugt 2 bis 6 copolymerisierbare, ethylenisch ungesättigte Doppelbindungen.

Besonders bevorzugt sind solche Verbindungen (G) mit einem Gehalt an ethylenisch ungesättigte Doppelbindungen von 0,1- 0,7 mol /100 g, ganz besonders bevorzugt 0,2 - 0,6 mol 1100 g.

Das zahlenmittlere Molekulargewicht Mₙ der Verbindungen (G) liegt, wenn nicht anders angegeben, bevorzugt unter 15000, besonders bevorzugt bei 300 -12000, ganz besonders bevorzugt bei 400 bis 5000 und insbesondere bei 500 - 3000 g/mol (bestimmt durch Gelpermeationschromatographie mit Polystyrol als Standard und Tetrahydrofuran als Elutionsmittel).

Als (Meth)acrylatverbindungen genannt seien (Meth)acrylsäureester und insbesondere Acrylsäureester sowie Vinylether von mehrfunktionellen Alkoholen, insbesondere solchen, die neben den Hydroxylgruppen keine weiteren funktionellen Gruppen oder allenfalls Ethergruppen enthalten. Beispiele solcher Alkohole sind z.B. bifunktionelle Alkohole, wie Ethylenglykol, Propylenglykol und deren höher kondensierte Vertreter, z.B. wie Diethylenglykol, Triethylenglykol, Dipropylenglykol, Tripropylenglykol etc., 1,2-, 1,3-oder 1,4-Butandiol, 1,5-Pentandiol, 1,6-Hexandiol, 3-Methyl-1,5-pentandiol, Neopentylglykol, alkoxylierte phenolische Verbindungen, wie ethoxylierte bzw. propoxylierte Bisphenole, 1,2-, 1,3- oder 1,4-Cyclohexandimethanol, trifunktionelle und höherfunktionelle Alkohole, wie Glycerin, Trimethylolpropan, Butantriol, Trimethylolethan, Pentaerythrit, Ditrimethylolpropan, Dipentaerythrit, Sorbit, Mannit und die entsprechenden alkoxylierten, insbesondere ethoxylierten und/oder propoxylierten Alkohole.

Die Alkoxylierungsprodukte sind in bekannter Weise durch Umsetzung der vorstehenden Alkohole mit Alkylenoxiden, insbesondere Ethylen- oder Propylenoxid, erhältlich. Vorzugsweise beträgt der Alkoxylierungsgrad je Hydroxylgruppe 0 bis 10, d.h. 1 mol Hydroxylgruppe kann mit bis zu 10 mol Alkylenoxiden alkoxyliert sein.

Als (Meth)acrylatverbindungen seien weiterhin Polyester(meth)acrylate genannt, wobei es sich um die (Meth)acrylsäureester oder Vinylether von Polyesterolen handelt, sowie Urethan-, Epoxid- oder Melamin(meth)acrylate.

Urethan(meth)acrylate sind z.B. erhältlich durch Umsetzung von Polyisocyanaten mit Hydroxyalkyl(meth)acrylaten und gegebenenfalls Kettenverlängerungsmitteln wie Diolen, Polyolen, Diaminen, Polyaminen oder Dithiolen oder Polythiolen.

Die Urethan(meth)acrylate haben vorzugsweise ein zahlenmittleres Molgewicht Mₙ von 500 bis 20 000, insbesondere von 750 bis 10 000 besonders bevorzugt 750 bis 3000 g/mol (bestimmt durch Gelpermeationschromatographie mit Polystyrol als Standard).

Die Urethan(meth)acrylate haben vorzugsweise einen Gehalt von 1 bis 5, besonders bevorzugt von 2 bis 4 Mol (Meth)acrylgruppen pro 1000 g Urethan(meth)acrylat.

Epoxid(meth)acrylate sind erhältlich durch Umsetzung von Epoxiden mit (Meth)acrylsäure. Als Epoxide in Betracht kommen z.B epoxidierte Olefine oder Glycidylether, z.B. Bisphenol-A-diglycidylether oder aliphatische Glycidylether, wie Butandioldiglycidether.

Melamin(meth)acrylate sind erhältlich durch Umsetzung von Melamin mit (Meth)acrylsäure oder deren Ester.

Die Epoxid(meth)acrylate und Melamin(meth)acrylate haben vorzugsweise ein zahlenmittleres Molgewicht Mₙ von 500 bis 20000, besonders bevorzugt von 750 bis 10000 g/mol und ganz besonders bevorzugt von 750 bis 3000 g/mol; der Gehalt an (Meth)acrylgruppen beträgt vorzugsweise 1 bis 5, besonders bevorzugt 2 bis 4 pro 1000 g Epoxid(meth)acrylat oder Melamin(meth)acrylat (bestimmt durch Gelpermeationschromatographie mit Polystyrol als Standard und Tetrahydrofuran als Elutionsmittel).

Weiterhin geeignet sind Carbonat(meth)acrylate, die im Mittel vorzugsweise 1 bis 5, insbesondere 2 bis 4, besonders bevorzugt 2 bis 3 (Meth)acrylgruppen und ganz besonders bevorzugt 2(Meth)acrylgruppen enthalten.

Das zahlungsmittlere Molekulargewicht Mₙ der Carbonat(meth)acrylate ist vorzugsweise kleiner 3000 g/mol, besonders bevorzugt kleiner 1500 g/mol, besonders bevorzugt kleiner 800 g/mol (bestimmt durch Gelpermeationschromatgraphie mit Polystyrol als Standard, Lösemittel Tetrahydrofuran).

Die Carbonat(meth)acrylate sind in einfacher Weise erhältlich durch Umesterung von Kohlensäureestern mit mehrwertigen, vorzugsweise zweiwertigen Alkoholen (Diolen, z.B. Hexandiol) und anschließende Veresterung der freien OH-Gruppen mit (Meth)-acrylsäure oder auch Umesterung mit (Meth)acrylsäureestern, wie es z.B. in EP-A 92 269 beschrieben ist. Erhältlich sind sie auch durch Umsetzung von Phosgen, Harnstoffderivaten mit mehrwertigen, z.B. zweiwertigen Alkoholen.

Als Reaktiwerdünner (Verbindungen (H)) kommen strahlungshärtbare, radikalisch oder kationisch polymerisierbare Verbindungen mit nur einer ethylenisch ungesättigten, copolymerisierbaren Gruppe in Betracht.

Genannt seien z.B. C₁-C₂₀-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen, α,β-ungesättigte Carbonsäuren und deren Anhydride und aliphatischen Kohlenwasserstoffen mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

α,β-Ungesättigte Carbonsäuren und deren Anhydride können beispielsweise sein Acrylsäure, Methacrylsäure, Fumarsäure, Crotonsäure, Itaconsäure, Maleinsäure oder Maleinsäureanhydrid, bevorzugt Acrylsäure.

Als vinylaromatische Verbindungen kommen z.B. Vinyltoluol, α-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C Atomen und einer oder zwei olefinischen Doppelbindungen seien Butadien, Isopren, sowie Ethylen, Propylen und Isobutylen genannt.

Weiterhin sind N-Vinylformamid, N-Vinylpyrrolidon sowie N-Vinylcaprolactam einsetzbar.

Als Photoinitiatoren (I) können dem Fachmann bekannte Photoinitiatoren verwendet werden, z.B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

In Betracht kommen z.B. Mono- oder Bisacylphosphinoxide Irgacure 819 (Bis(2,4,6-Trimethylbenzoyl)phenylphosphinoxid), wie sie z.B. in EP-A 7508, EP-A 57 474, DE-A 196 18 720, EP-A 495 751 oder EP-A 615 980 beschrieben sind, beispielsweise 2,4,6-Trimethylbenzoyldiphenylphosphinoxid (Lucirin^{®} TPO), Ethyl-2,4,6-trimethylbenzoylphenylphosphinat, Benzophenone, Hydroxyacetophenone, Phenylglyoxylsäure und ihre Derivate oder Gemische dieser Photoinitiatoren. Als Beispiele seien genannt Benzophenon, Acetophenon, Acetonaphthochinon, Methylethylketon, Valerophenon, Hexanophenon, α-Phenylbutyrophenon, p-Morpholinopropiophenon, Dibenzosuberon, 4-Morpholinobenzophenon, 4-Morpholinodeoxybenzoin, p-Diacetylbenzol, 4-Aminobenzophenon, 4'-Methoxyacetophenon, β-Methylanthrachinon, *tert*-Butylanthrachinon, Anthrachinoncarbonysäureester, Benzaldehyd, α-Tetralon, 9-Acetylphenanthren, 2-Acetylphenanthren, 10-Thioxanthenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Di-*iso*-propylthioxanthon, 2,4-Dichlorthioxanthon, Benzoin, Benzoin-*iso*-butylether, Chloroxanthenon, Benzointetrahydropyranylether, Benzoin-methylether, Benzoin-ethylether, Benzoin-butylether, Benzoin-iso-propylether, 7-H-Benzoin-methylether, Benz[de]anthracen-7-on, 1-Naphthaldehyd, 4,4'-Bis(dimethylamino)benzophenon, 4-Phenylbenzophenon, 4-Chlorbenzophenon, Michlers Keton, 1-Acetonaphthon, 2-Acetonaphthon, 1-Benzoylcyclohexan-1-ol, 2-Hydroxy-2,2-dimethylacetophenon, 2,2-Dimethoxy-2-phenylacetophenon, 2,2-Diethoxy-2-phenylacetophenon, 1,1-Dichloracetophenon, 1-Hydroxyacetophenon, Acetophenondimethylketal, o-Methoxybenzophenon, Triphenylphosphin, Tri-o-Tolylphosphin, Benz[a]anthracen-7,12-dion, 2,2-Diethoxyacetophenon, Benzilketale, wie Benzildimethylketal, 2-Methyl-1-[4-(methylthio)phenyl]-2-morpholinopropan-1-on, Anthrachinone wie 2-Methylanthrachinon, 2-Ethylanthrachinon, 2-tert-Butylanthrachinon, 1-Chloranthrachinon, 2-Amylanthrachinon und 2,3-Butandion.

Geeignet sind auch nicht- oder wenig vergilbende Photoinitiatoren vom Phenylglyoxalsäureestertyp, wie in DE-A 198 26 712, DE-A 199 13 353 oder WO 98/33761 beschrieben.

Unter den genannten Photoinitiatoren sind Phosphinoxide, α-Hydroxyketone und Benzophenone bevorzugt.

Insbesondere können auch Gemische verschiedener Photoinitiatoren verwendet werden.

Die Photoinitiatoren können allein oder in Kombination mit einem Photopolymerisationspromotor, z.B. vom Benzoesäure-, Amin- oder ähnlichem Typ verwendet werden.

Als weitere lacktypische Additive (J) können beispielsweise Antioxidantien, Oxidationsinhibitoren, Stabilisatoren, Aktivatoren (Beschleuniger), Füllmittel, Pigmente, Farbstoffe, Entgasungsmittel, Glanzmittel, antistatische Agentien, Flammschutzmittel, Verdicker, thixotrope Agentien, Verlaufshilfsmittel, Bindemittel, Antischaummittel, Duftstoffe, oberflächenaktive Agentien, Viskositätsmodifikatoren, Weichmacher, Plastifizierer, klebrigmachende Harze (Tackifier), Chelatbildner oder Verträglichkeitsmittel (compatibilizer) verwendet werden.

Als Beschleuniger für die thermische Nachhärtung kann z.B. Zinnoctoat, Zinkoctoat, Dibutylzinnlaureat oder Diaza[2.2.2]bicyclooctan verwendet werden.

Weiterhin können ein oder mehrere photochemisch und/oder thermisch aktivierbare Initiatoren zugesetzt werden, z.B. Kaliumperoxodisulfat, Dibenzoylperoxid, Cyclohexanonperoxid, Di-tert.-Butylperoxid, Az--obis-iso-butyronitril, Cyclohexylsulfonylacetylperoxid, Di-iso-propylpercarbonat, tert-Butylperoktoat oder Benzpinakol, sowie beispielsweise solche thermisch aktivierbare Initiatoren, die eine Halbwertszeit bei 80°C von mehr als 100 Stunden aufweisen, wie Di-t-Butylperoxid, Cumolhydroperoxid, Dicumylperoxid, t-Butylperbenzoat, silylierte Pinakole, die z. B. unter dem Handelsnamen AD-DID 600 der Firma Wacker kommerziell erhältlich sind oder Hydroxylgruppen-haltige Amin-N-Oxide, wie 2,2,6,6-Tetramethylpiperidin-N-oxyl, 4-Hydroxy-2,2,6,6-Tetramethylpiperidin-N-oxyl etc.
Weitere Beispiele geeigneter Initiatoren sind in "Polymer Handbook", 2. Aufl., Wiley & Sons, New York beschrieben.

Als Verdicker kommen neben radikalisch (co)polymerisierten (Co)Polymerisaten, übliche organische und anorganische Verdicker wie Hydroxymethylcellulose oder Bentonite in Betracht.

Als Chelatbildner können z.B. Ethylendiaminessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil^{®} der Fa. Degussa, Kieselerde, Talkum, Aluminiumsilikate, Magnesiumsilikate, Calciumcarbonate etc.

Geeignete Stabilisatoren umfassen typische UV-Absorber wie Oxanilide, Triazine und Benzotriazol (letztere erhältlich als Tinuvin^{®} -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat, eingesetzt werden. Stabilisatoren werden üblicherweise in Mengen von 0,1 bis 5,0 Gew.%, bezogen auf die in der Zubereitung enthaltenen festen Komponenten, eingesetzt.

Weiterhin geeignete Stabilisatoren sind beispielsweise N-Oxyle, wie z.B. 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6;6-tetramethyl-piperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl, Phenole und Naphthole, wie z.B. p-Aminophenol, p-Nitrosophenol, 2-*tert.*-Butylphenol, 4-*tert*.-Butylphenol, 2,4-di-*tert*.-Butylphenol, 2-Methyl-4-tert.-Butylphenol, 4-Methyl-2,6-fert.-Butylphenol (2,6-*tert:* Butyl-p-Kresol) oder 4-*tert.-*Butyl-2,6-dimethylphenol, Chinone, wie z.B. Hydrochinon oder Hydrochinonmonomethylether, aromatische Amine, wie z.B. N,N-Diphenylamin, N-Nitroso-diphenylamin, Phenylendiamine, wie z.B. N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, Hydroxylamine, wie z.B. N,N-Diethylhydroxylamin, Harnstoffderivate, wie z.B. Harnstoff oder Thioharnstoff, phosphorhaltige Verbindungen, wie z.B. Triphenylphosphin, Triphenylphosphit oder Triethylphosphit oder schwefelhaltige Verbindungen, wie z.B. Diphenylsulfid oder Phenothiazin.

Typische Zusammensetzungen für strahlungshärtbare Massen sind beispielsweise
(F) 20 -100 Gew%, bevorzugt 40 - 90, besonders bevorzugt 50 - 90 und insbesondere 60 - 80 Gew%,
(G) 0 - 60 Gew%, bevorzugt 5 - 50, besonders bevorzugt 10 - 40 und insbesondere 10 - 30 Gew%,
(H) 0-50 Gew%, bevorzugt 5-40, besonders bevorzugt 6 - 30 und insbesondere 10 - 30 Gew%,
(I) 0 - 20 Gew%, bevorzugt 0,5 -15, besonders bevorzugt 1-10 und insbesondere 2 - 5 Gew% sowie
(J) 0 - 50 Gew%, bevorzugt 2 - 40, besonders bevorzugt 3 - 30 und insbesondere 5 - 20 Gew%,
mit der Maßgabe, daß (F), (G), (H), (I) und (J) zusammen 100 Gew% ergeben.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die gegebenenfalls enthaltenen flüchtigen Bestandteile der Bechichtungsmasse, gegebenenfalls unter Erhitzen, entfernt. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen. Die Beschichtungsstärke liegt in der Regel in einem Bereich von etwa 3 bis 1000 g/m² und vorzugsweise 10 bis 200 g/m².

Weiterhin wird ein Verfahren zum Beschichten von Substraten offenbart, bei dem man die Beschichtungsmasse auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur und anschließend bei Temperaturen bis zu 160°C, bevorzugt zwischen 60 und 160°C, thermisch behandelt.

Das Verfahren zum Beschichten von Substraten kann auch so durchgeführt werden, daß nach dem Aufbringen der Beschichtungsmasse zunächst bei Temperaturen bis zu 160°C, bevorzugt zwischen 60 und 160 °C, thermisch behandelt und anschließend mit Elektronenstrahlen oder UV Belichtung unter Sauerstoff oder bevorzugt unter Inertgas gehärtet wird.

Die Härtung der auf dem Substrat gebildeten Filme kann gewünschtenfalls ausschließlich thermisch erfolgen. Im allgemeinen härtet man die Beschichtungen jedoch sowohl durch Bestrahlung mit energiereicher Strahlung als auch thermisch.

Die Härtung kann auch zusätzlich oder anstelle der thermischen Härtung durch NIR-Strahlung erfolgen, wobei als NIR-Strahlung hier elektromagnetische Strahlung im Wellenlängenbereich von 760 nm bis 2,5 µm, bevorzugt von 900 bis 1500 nm bezeichnet ist.

Gegebenenfalls kann, wenn mehrere Schichten des Beschichtungsmittels übereinander aufgetragen werden, nach jedem Beschichtungsvorgang eine thermische, NIR und/oder Strahlungshärtung erfolgen.

Als Strahlungsquellen für die Strahlungshärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ=200 bis 700 nm strahlt, besonders bevorzugt von λ=200 bis 500 nm und ganz besonders bevorzugt λ=250 bis 400 nm, oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm².

Selbstverständlich sind auch mehrere Strahlungsquellen für die Härtung einsetzbar, z.B. zwei bis vier.
Diese können auch in jeweils unterschiedlichen Wellenlängebereichen strahlen.

Die Bestrahlung kann gegebenenfalls auch unter Ausschluß von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase. Desweiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE-A1 199 57 900 beschrieben ist.

Ein weiterer Gegenstand der Erfindung ist ein Verfahren zur Beschichtung von Substraten, wobei man
i) ein Substrat mit einer Beschichtungsmasse, wie zuvor beschrieben, beschichtet,
ii) flüchtige Bestandteile der Beschichtungsmasse zur Filmbildung unter Bedingungen entfernt, bei denen der Photoinitiator (I) im wesentlichen noch keine freien Radikale ausbildet,
iii) gegebenenfalls den in Schritt ii) gebildeten Film mit energiereicher Strahlung bestrahlt, wobei der Film vorgehärtet wird, und anschließend gegebenenfalls den mit dem vorgehärteten Film beschichteten Gegenstand mechanisch bearbeitet oder die Oberfläche des vorgehärteten Films mit einem anderen Substrat in Kontakt bringt,
iv) dem Film thermisch oder mit NIR-Strahlung endhärtet

Dabei können die Schritte iv) und iii) auch in umgekehrter Reihenfolge durchgeführt, d. h. der Film kann zuerst thermisch oder per NIR-Strahlung und dann mit energiereicher Strahlung gehärtet werden.

Weiterhin sind auch Substrat, beschichtet mit einer erfindungsgemäßen Mehrschichtlackierung Gegenstand der vorliegenden Erfindung.

Die Dicke einer solche wie beschrieben zu härtenden Schicht kann von 0,1 µm bis mehrere mm betragen, bevorzugt von 1 bis 2000 µm, besonders bevorzugt 5 bis 1000 µm, ganz besonders bevorzugt von 10 bis 500 µm und insbesondere von 10 bis 250 µm.

Besonders bevorzugt eignen sich die erfindungsgemäßen Beschichtungsmassen als oder in Außenbeschichtungen, also solche Anwendungen, die dem Tageslicht ausgesetzt sind, bevorzugt von Gebäuden oder Gebäudeteilen, Innenbeschichtungen, Straßenmarkierungen, Beschichtungen auf Fahrzeugen und Flugzeugen. Insbesondere werden die Beschichtungen als Holz-, Papier- oder Kunststoffbeschichtungen, beispielsweise für Parkett oder Möbel eingesetzt.

Mit Hilfe des erfindungsgemäßen Verfahrens ist die Herstellung teilveresterten (Meth)acrylsäureester (F) in hoher chemischer und Raum-Zeit-Ausbeute und unter milden Bedingungen mit guten Farbzahlen möglich. Durch die Verwendung von Schutzgruppen werden gezielt die gewünschten teilveresterten Produkte erhalten, die frei von Nebenprodukten sind.

Die folgenden Beispiele sollen die Eigenschaften der Erfindung erläutern, ohne sie aber einzuschränken.

### Beispiele

Als "Teile" seien in dieser Schrift, wenn nicht anders angegeben, "Gewichtsteile" verstanden.

### Beispiel 1: Isopropyliden-Glycerin + Ethylacrylat (Variation des Lösungsmittels)

Es wurden 5 mmol 2,3-Isopropyliden-Glycerin, 50 mmol Ethylacrylat, 10 ml Lösungsmittel (LM) und 100 mg Novozym 435 für 24 h bei 40 °C geschüttelt.
Das Reaktionsgemisch wurde filtriert und der Umsatz zum 2,3-Isopropyliden-Glycerinmonoacrylat gaschromatografisch ermittelt.

| Lösungsmittel | Umsatz [%] |
|---|---|
| ohne LM | 74 |
| Aceton | 68 |
| 1,4-Dioxan | 74 |
| MTBE | 74 |
| Toluol | 74 |
| Acetonitril | 74 |
| THF | 71 |

| | |
|---|---|
| MTBE: tert-Butyl-methylether | |

### Beispiel 2: Isopropyliden-Glycerin + Alkylacrylat (Variation des Alkylacrylats)

Es wurden 5 mmol 2,3-lyopropyliden-Glycerin (IPG), 25-100 mmol Alkylacrylat und 100 mg Novozym 435 für 24 h bei 20, 40 oder 60 °C geschüttelt. Das Reaktionsgemisch wurde filtriert und der Umsatz zum 2,3-Isopropyliden-Glycerinmonoacrylat gaschromatografisch ermittelt.
bei 20 °C

| Acrylat : IPG [mol/mol] | Umsatz [%] mit Methylacrylat | Umsatz [%] mit Ethylacrylat | Umsatz [%] mit Butylacrylat |
|---|---|---|---|
| 5 : 1 | 58 | 65 | 61 |
| 7 : 1 | 61 | 69 | 67 |
| 10 : 1 | 67 | 74 | 71 |
| 10 : 1 (72 h) | 67 | 74 | 71 |
| 20 : 1 | 76 | 83 | 80 |

bei 40 °C

| Acrylat : IPG [mol/mol] | Umsatz [%] mit Methylacrylat | Umsatz [%] mit Ethylacrylat | Umsatz [%] mit Butylacrylat |
|---|---|---|---|
| 5 : 1 | 60 | 66 | 64 |
| 10 : 1 | 71 | 76 | 75 |
| 10 : 1 (72 h) | 69 | 75 | 73 |
| 20 : 1 | 79 | 85 | 84 |

bei 60 °C

| Acrylat : IPG [mol/mol] | Umsatz [%] mit Methylacrylat | Umsatz [%] mit Ethylacrylat | Umsatz [%] mit Butylacrylat |
|---|---|---|---|
| 5 : 1 | 61 | 67 | 66 |
| 10:1 | 72 | 78 | 76 |
| 10 : 1(72 h) | 72 | 78 | 77 |
| 20:1 | 81 | 86 | 85 |

### Beispiel 3: Isopropyliden-Glycerin + Methylacrylat

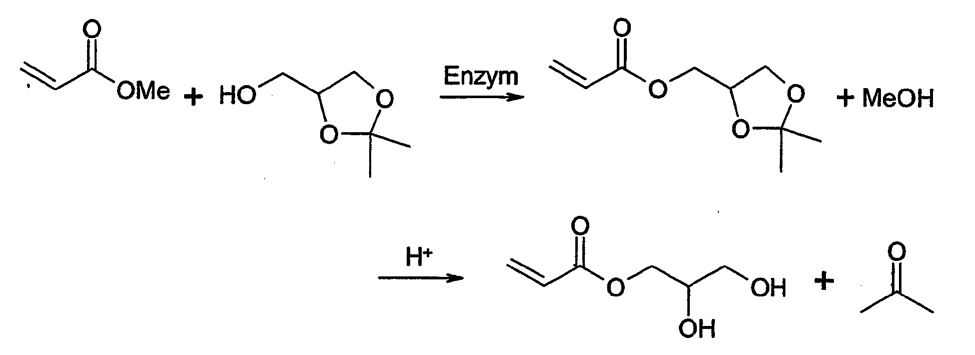

Es wurden 4,0 mol (528,8 g) 2,3-Isopropyliden-Glycerin, 20,0 mol (1722 g) Methylacrylat, 86 mg Phenothiazin, 344 mg p-Methoxyphenol, 800 g Molsieb (5 Å) und 80,0 g Novozym 435 für 24 h bei Raumtemperatur in einem Rundkolben gerührt.
Das Reaktionsgemisch wurde filtriert und der Überschuß Methylacrylat am Rotationsverdampfer entfernt. Der Ansatz wurde zweimal durchgeführt mit reproduzierbaren Umsätzen.

| Ansatz | Umsatz [%] | Ausbeute [g] |
|---|---|---|
| A | 93 | 674,6 |
| B | 94 | 724,1 |

Zur Hydrolyse der Isopropylidengruppe wurden 680 g 2,3-Isopropyliden-Glycerinmonoacrylat mit 680 g Wasser und 68 g stark saurem Ionenaustauscher (Dowex 50 Wx8 H⁺-Form) 24 h bei Raumtemperatur gerührt. Der Ionenaustauscher wurde abfiltriert und das entstandene Aceton am Rotationsverdampfer bei 20-30 °C im Vakuum entfernt.

Die entstandene wässerige Lösung von Glycerinmonoacrylat kann direkt weiter verwendet werden zur Copolymerisation. Laut GC-Analytik bestehend die organischen Anteile der Lösung aus 94,3 % Glycerinmonoacrylat, 5,2 % Glycerin, 0,5 % 2,3-lsopropyliden-Glycerinmonoacryat.

Alternativ kann ein wasserfreies Produkt hergestellt werden, indem die wässerige Lösung mit Essigester extrahiert, über Natriumsulfat getrocknet und am Rotationsverdampfer wieder vom Essigester befreit wird. Das wasserfreie, farblose Öl besteht laut GC-Analytik zu 97,1 % aus Glycerinmonoacrylat, 1,9 %Glycerin, 0,6 % 2,3-Isopropyliden-Glycerinmonoacrylat und 0,3 % 2,3-Isopropyliden-Glycerin.

### Beispiel 4: 2,3-Isopropyliden-Glycerin + Methylacrylat

In einem Rundkolben wurden 0,1 mol (13,2 g) 2,3-Isopropyliden-Glycerin, 1 mol (86,1 g) Methylacrylat und 650 mg Novozym 435 bei 60 °C im Vakuum (450-470 mbar) gerührt. Der Dampf (Methylacrylat + Methanol) wurde in einem Rohr in einen Kühler geleitet. Das Kondensat tropfte über einen mit 20 g Molsieb (5 Å) gefüllten Tropftrichter zurück in den Reaktionsansatz. Nach 6 h wurde eine Probe aus dem Ansatz entnommen und der Umsatz mittels GC zu 99,5 % bestimmt.

### Beispiel 5: 2,3-Isopropyliden-Glycerin + Methylmethacrylat

Es wurden 5 mmol 2,3-Isopropyliden-Glycerin (IPG), 10-50 mmol Methylmethacrylat (MMA), 1,0 g Molsieb (5 Å) und 100 mg Novozym 435 für 24 h bei 20 oder 40 °C geschüttelt.
Das Reaktionsgemisch wurde filtriert und der Umsatz zum 2,3-Isopropyliden-Glycerinmonomethacrylat gaschromatografisch ermittelt.

| MMA : IPG [mol:mol] | Umsatz [%] bei 20°C | Umsatz [%] bei 40 °C |
|---|---|---|
| 2 : 1 | 78 | 91 |
| 3 : 1 | 78 | 89 |
| 5 : 1 | 90 | 90 |
| 7 : 1 | 92 | 94 |
| 10: 1 | 91 | 95 |

### Beispiel 6: Isopropylidengeschütztes-Trimethylolpropan + Methylacrylat

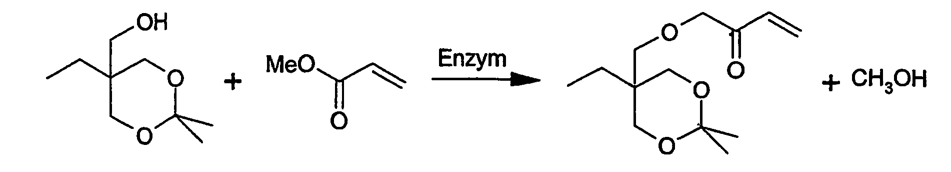

Es wurden 5 mmol Isopropyliden-Trimethylolpropan (IPT), 10-50 mmol Methylacrylat (MA), 1,0 g Molsieb (5 Å), evtl. 10 ml Methyl-*tert*-butylether (MTBE) und 100 mg Novozym 435 für 24 oder 72 h bei 20, 40 oder 60 °C geschüttelt.
Das reaktionsgemisch wurde filtriert und der Umsatz zum Isopropyliden-Trimethylolpropanmonoacrylat gaschromatografisch ermittelt.

**Reaktionen ohne MTBE**

| MA : IPT [mol:mol] | Umsatz [%] bei 20°C | Umsatz [%] bei 40 °C | Umsatz [%] bei 60°C |
|---|---|---|---|
| 2 : 1 (24 h) | 14 | 20 | 19 |
| 5 : 1 (24 h) | 14 | 16 | 22 |
| 10 : 1 (24 h) | 11 | 16 | 16 |
| 10 : 1 (72 h) | 14 | 21 | - |

**Reaktionen mit MTBE**

| MA: IPT [mol:mol] | Umsatz [%] bei 20 °C | Umsatz [%] bei 40 °C |
|---|---|---|
| 2 : 1 (24 h) | 10 | 21 |
| 5 : 1 (24 h) | 11 | 19 |
| 10 : 1 (24 h) | 9 | 17 |
| 10 : 1(72 h) | 8 | 21 |

### Beispiel 7: Isopropyliden-Glucofuranose + Methylacrylat

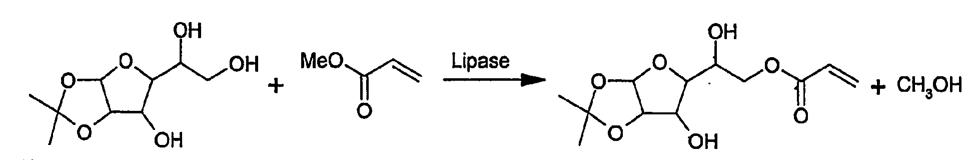

Es wurden 5 mmol Isopropyliden-Glucofuranose (IP-Glu), 50 mmol Methylacrylat (MA), evtl. 1,0 g Molsieb (5 Å), evtl. 10 ml Lösungsmittel und 100 mg Novozym 435 für 24 h bei 40 °C geschüttelt.
Das Reaktionsgemisch wurde filtriert und der Umsatz zum Isopropyliden-Glucofuranosemonoacrylat gaschromatografisch ermittelt.

| Lösungsmittel | Umsatz [%] ohne Molsieb | Umsatz [%] mit Molsieb |
|---|---|---|
| ohne LM | 53 | 85 |
| Aceton | 51 | 73 |
| 1,4-Dioxan | 54 | 80 |
| THF | 52 | 68 |

## Patentansprüche

1. Verfahren zur Herstellung von teilveresterten (Meth)acrylsäureestern **dadurch gekennzeichnet, daß** man in einem Schritt c) Polyalkohole (C), in denen mindestens zwei Hydroxygruppen gemeinsam in einer Acetal- oder Ketalgruppe verbunden sind,
in Gegenwart mindestens eines Enzyms (E) mit (Meth)acrylsäure verestert oder in Gegenwart mindestens eines Enzyms (E) mit mindestens einem (Meth)acrylat (D) umestert, wobei das (Meth)acrylat (D) ein Ester von (Meth)acrylsäure mit einem gesättigten Alkohol ist.

2. Verfahren gemäß Anspruch 1, **dadurch gekennzeichnet, dass** es sich bei der Verbindung (D) um einen gesättigten C₁-C₁₀-Alkylester der (Meth)acrylsäure handelt.

3. Verfahren gemäß Anspruch 1 oder 2, **dadurch gekennzeichnet, daß** die Polyalkohole (C) erhältlich sind durch
a) Umsetzung eines Aldehyds oder Ketons (A) mit einem Polyol (B) und
b) gegebenenfalls Aufreinigung des aus a) erhältlichen Reaktionsgemisches, worin
Aldehyde oder Ketone (A) solche der Formel I sind,
R¹-C(=O)-R² (I),
worin
R¹ und R² unabhängig voneinander Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können oder R¹ und R² gemeinsam mit dem Carbonylkohlenstoff einen vier- bis zwölfgliedrigen Ring bilden können,
und Polyole (B) Polyole mit 3 bis 10 Hydroxygruppen sind.

4. Verfahren nach einem der Ansprüche 1 bis 3, **dadurch gekennzeichnet, daß** die Polyalkohole (C) eine in Position 2 mono- oder disubstituierte 1,3-Dioxolan- oder 1,3-Dioxanstruktur enthalten.

5. Verfahren nach einem der vorstehenden Ansprüche, **dadurch gekennzeichnet, daß** die Polyalkohole (C) ausgewählt sind aus der Gruppe 4-Hydroxymethyl-2,2-di-methyl-1,3-dioxolan, 4-Hydroxymethyl-2-methyl-1,3-dioxolan, 4-Hydroxymethyl-2,2-diethyl-1,3-dioxolan, 4-Hydroxymethyl-2-*tert*-butyl-1,3-dioxolan, 4-Hydroxy-methyl-2-phenyl-1,3-dioxolan, 5-Ethyl-5-hydroxymethyl-2,2-dimethyl-1,3-dioxan, 5-Ethyl-5-hydroxymethyl-2-methyl-1,3-dioxan, 5-Ethyl-5-hydroxymethyl-2,2-diethyl-1,3-dioxan, 5-Ethyl-5-hydroxymethyl-2-tert-butyl-1,3-dioxan, 5-Ethyl-5-hydroxymethyl-2-phenyl-1,3-dioxan, 1,2-O-Isopropyliden-a-D-glucofuranose, 2,3-O-Isopropyliden-threitol (= 2,2-Dimethyl-1,3-dioxolan-4,5-dimethanol) und 5,5-Bis(hydroxymethyl)-2,2-dimethyl-1,3-dioxan.

6. Verfahren nach einem der vorstehenden Ansprüche, **dadurch gekennzeichnet, daß** das Reaktionsgemisch aus Schritt c) in einem Schritt d) aufgereinigt wird.

7. Verfahren nach einem der vorstehenden Ansprüche, **dadurch gekennzeichnet, daß** das Reaktionsgemisch aus Schritt c) und oder Schritt d) in einem Schritt e) entschützt und gegebenenfalls in einer Stufe f) aufgereinigt wird.

8. Verfahren nach Anspruch 7, **dadurch gekennzeichnet, dass** man den Schritt e) in Gegenwart von Salzsäure, Schwefelsäure, Salpetersäure, Phosphorsäure, para-Toluolsulfonsäure, Benzolsulfonsäure, Methansulfonsäure, Trifluormethansulfonsäure, mineralischen Tonen, sauren Ionentauschern oder katalysiert durch Enzyme unter Zugabe von bis zu 20 Gew.% Wasser durchführt.

9. Verfahren nach Anspruch 7 oder 8, **dadurch gekennzeichnet, dass** man den Schritt e) bei einer Temperatur von 20 bis 150°C durchführt.

10. Verfahren nach einem der vorstehenden Ansprüche, **dadurch gekennzeichnet, daß** das Enzym (E) ausgewählt ist unter Esterasen (E.C. 3.1.-.-), Lipasen (E.C. 3.1.1.3), Glykosylasen (E.C. 3.2.-.-) und Proteasen (E.C. 3.4.-.-).

## Claims

1. A process for preparing partial (meth)acrylic esters which comprises a step c) of subjecting polyalcohols (C) in which at least two hydroxyl groups together are joined in an acetal group or ketal group
to esterification with (meth)acrylic acid in the presence of at least one enzyme (E) or to transesterification with at least one (meth)acrylate (D) in the presence of at least one enzyme (E), the (meth)acrylate (D) being an ester of (meth)acrylic acid with a saturated alcohol.

2. The process according to claim 1, wherein the compound (D) is a saturated C₁-C₁₀-alkyl (meth)acrylate.

3. The process according to claim 1 or 2, wherein the polyalcohols (C) are obtainable by
a) reacting an aldehyde or ketone (A) with a polyol (B) and
b) where appropriate, purifying the reaction mixture obtainable from a),
where
aldehydes or ketones (A) are of the formula I
R¹-C(=O)-R² (I),
where
R¹ and R² independently of one another are hydrogen, C₁-C₁₈ alkyl, C₂-C₁₈ alkyl uninterrupted or interrupted by one or more oxygen and/or sulfur atoms and/or by one or more substituted or unsubstituted imino groups, or are C₂-C₁₈ alkenyl, C₆-C₁₂ aryl, C₅-C₁₂ cycloalkyl or a five- or six-membered, oxygen-, nitrogen- and/or sulfur-containing heterocycle, it being possible for the specified radicals to be substituted in each case by aryl, alkyl, aryloxy, alkyloxy, heteroatoms and/or heterocycles or for R¹ and R² together with the carbonyl carbon to form a four- to twelve-membered ring,
and polyols (B) are polyols having from 3 to 10 hydroxyl groups.

4. The process according to any of claims 1 to 3, wherein the polyalcohols (C) comprise a 1, 3-dioxolane or 1,3-dioxane structure which is mono-or disubstituted in position 2.

5. The process according to any of the preceding claims, wherein the polyols (C) are selected from the group consisting of 4-hydroxymethyl-2,2-dimethyl-1,3-dioxolane, 4-hydroxymethyl-2-methyl-1,3-dioxolane, 4-hydroxymethyl-2,2-diethyl-1,3-dioxolane, 4-hydroxymethyl-2-tert-butyl-1,3-dioxolane, 4-hydroxymethyl-2-phenyl-1,3-dioxolane, 5-ethyl-5-hydroxymethyl-2,2-dimethyl-1,3-dioxane, 5-ethyl-5-hydroxymethyl-2-methyl-1,3-dioxane, 5-ethyl-5-hydroxymethyl-2,2-diethyl-1,3-dioxane, 5-ethyl-5-hydroxymethyl-2-tert-butyl-1,3-dioxane, 5-ethyl-5-hydroxymethyl-2-phenyl-1,3-dioxane, 1,2-O-isopropylidene-□-D-glucofuranose, 2,3-O-isopropylidene-threitol (= 2,2-dimethyl-1,3-dioxolane-4,5-dimethanol), and 5,5-bis(hydroxymethyl)-2,2-dimethyl-1,3-dioxane.

6. The process according to any of the preceding claims, wherein the reaction mixture from step c) is purified in a step d).

7. The process according to any of the preceding claims, wherein the reaction mixture from step c) and/or step d) is deprotected in a step e) and, where appropriate, purified in a stage f).

8. The process according to claim 7, wherein step e) is conducted in the presence of hydrochloric acid, sulfuric acid, nitric acid, phosphoric acid, paratoluenesulfonic acid, benzenesulfonic acid, methanesulfonic acid, trifluoromethanesulfonic acid, mineral clays, acidic ion exchangers or catalyzed by enzymes, with the addition of up to 20% by weight of water.

9. The process according to claim 7 or 8, wherein step e) is conducted at a temperature of from 20 to 150°C.

10. The process according to any of the preceding claims, wherein the enzyme (E) is selected from esterases (E.C. 3.1.-.-), lipases (E.C. 3.1.1.3), glycosylases (E.C. 3.2.-.-), and proteases (E.C. 3.4.-.-).

## Revendications

1. Procédé de préparation d'esters d'acide (méth)acrylique partiellement estérifiés, **caractérisé en ce que**, dans une étape c), on estérifie des polyalcools (C), dans lesquels au moins deux groupes hydroxy sont reliés conjointement dans un groupe acétal ou cétal, avec de l'acide {méth}acrylique, en présence d'au moins une enzyme (E), ou on les transestérifie avec au moins un (méth)acrylate (D), en présence d'au moins une enzyme (E), le {méth}acrylate (D) étant un ester d'acide (méth)acrylique avec un alcool saturé.

2. Procédé suivant la revendication 1, **caractérisé en ce que** le composé (D) est un ester alkylique en C₁-C₁₀ saturé de l'acide (méth) acrylique.

3. Procédé suivant la revendication 1 ou 2, **caractérisé en ce que** les polyalcools (C) peuvent être obtenus par
a) réaction d'un aldéhyde ou d'une cétone (A) avec un polyol (B), et
b) éventuellement purification du mélange réactionnel obtenu dans a),
les aldéhydes ou cétones (A) étant ceux de la formule I :
R¹-C(=O) -R² (I)
dans laquelle R¹ et R² représentent, indépendamment l'un de l'autre, de l'hydrogène, un groupe alkyle en C₁-C₁₈, alkyle en C₂-C₁₈ éventuellement interrompu par un ou plusieurs atomes d'oxygène et/ou de soufre et/ou par un ou plusieurs groupes imino substitués ou non substitués, alcényle en C₂-C₁₈, aryle en C₆-C₁₂, cycloalkyle en C₅-C₁₂ ou un hétérocycle pentagonal à hexagonal présentant des atomes d'oxygène, d'azote et/ou de soufre, les radicaux cités pouvant chacun être substitué par des groupes aryle, alkyle, aryloxy, alkyloxy, des hétéroatomes et/ou des hétérocycles, ou R¹ et R² pouvant former conjointement avec le carbone du carbonyle un noyau à quatre jusqu'à douze termes, et les polyols (B) sont des polyols comportant 3 à 10 groupes hydroxy.

4. Procédé suivant l'une des revendications 1 à 3, **caractérisé en ce que** les polyalcools (C) contiennent une structure de 1,3-dioxolane ou de 1,3-dioxanne monosubstitué ou disubstitué en position 2.

5. Procédé suivant l'une des revendications précédentes, **caractérisé en ce que** les polyalcools (C) sont choisis parmi le groupe du 4-hydroxyméthyl-2,2-diméthyl-1,3-dioxolane, du 4-hydroxyméthyl-2-méthyl-1,3-dioxolane, du 4-hydroxyméthyl-2,2-diéthyl-1,3-dioxolane, du 4-hydroxyméthyl-2-tert-butyl-1,3-dioxolane, du 4-hydroxyméthyl-2-phényl-1,3-dioxolane, du 5-éthyl-5-hydroxyméthyl-2,2-diméthyl-1,3-dioxanne, du 5-éthyl-5-hydroxyméthyl-2-méthyl-1,3-dioxanne, du 5-éthyl-5-hydroxyméthyl-2,2-diéthyl-1,3-dioxanne, du 5-éthyl-5-hydroxyméthyl-2-tert-butyl-1,3-dioxanne, du 5-éthyl-5-hydroxyméthyl-2-phényl-1,3-dioxanne, du 1,2-O-isopropylidène-α-D-glucofuranose, du 2,3-O-isopropylidènethréitol (= 2,2-diméthyl-1,3-dioxolane-4,5-diméthanol) et du 5,5-bis(hydroxyméthyl)-2,2-diméthyl-1,3-dioxanne.

6. Procédé suivant l'une des revendications précédentes, **caractérisé en ce que** le mélange réactionnel de l'étape c) est purifié dans une étape d) .

7. Procédé suivant l'une des revendications précédentes, **caractérisé en ce que** le mélange réactionnel de l'étape c) et/ou de l'étape d) est déprotégé dans une étape e) et éventuellement purifié dans une étape f).

8. Procédé suivant la revendication 7, **caractérisé en ce qu'**on effectue l'étape e) en présence d'acide chlorhydrique, d'acide sulfurique, d'acide nitrique, d'acide phosphorique, d'acide para-toluènesulfonique, d'acide benzènesulfonique, d'acide méthanesulfonique, d'acide trifluorométhanesulfonique, d'argiles minérales, d'échangeurs d'ions acides ou d'une manière catalysée par des enzymes, avec addition de jusqu'à 20 % en poids d'eau.

9. Procédé suivant la revendication 7 ou 8, **caractérisé en ce qu'**on effectue l'étape e) à une température de 20 à 150°C.

10. Procédé suivant l'une des revendications précédentes, **caractérisé en ce que** l'enzyme (E) est choisie parmi des estérases (E.C. 3.1.-.-), des lipases (E.C. 3.1.1.3), des glycosylases (E.C. 3.2.-.-) et des protéases (E.C. 3.4.-.-).
